# EUROPEAN PATENT APPLICATION

(11) **EP 3 231 420 A1**
(43) Date of publication of application: **18.10.2017**
(21) Application number: 16157804.2
(22) Date of filing: 29.02.2016
(51) Int. Cl.: A61K 31/00

(54) **ABUSE-DETERRENT PHARMACEUTICAL COMPOSITIONS**

(71) Applicant: G.L. Pharma GmbH, 8502 Lannach (AT)
(72) Inventor: The designation of the inventor has not yet been filed
(74) Representative: Sonn & Partner Patentanwälte

(57) **Abstract**

Disclosed are pharmaceutical compositions comprising a drug with a laccase-reactive functional group and a laccase (EC 1.10.3.2) and methods for manufacturing such compositions.

## Description

The present application relates to abuse-deterrent pharmaceutical compositions comprising opioids.

Prescription opioid products are an important component of modern pain management. However, abuse and misuse of these products have created a serious and growing public health problem. One potentially important step towards the goal of creating safer opioid analgesics has been the development of opioids that are formulated to deter abuse. The development of these products is considered e.g. by the US FDA as a high public health priority (see "Abuse-Deterrent Opioids - Evaluation and Labeling"; Guidance for Industry FDA April 2015, FDA; "FDA Guidance").

Because opioid products are often manipulated for purposes of abuse by different routes of administration or to defeat extended-release (ER) properties, most abuse-deterrent technologies developed to date are intended to make manipulation more difficult or to make abuse of the manipulated product less attractive or less rewarding. It should be noted that these technologies have not yet proven successful at deterring the most common form of abuse-swallowing a number of intact capsules or tablets to achieve a feeling of euphoria. Moreover, the fact that a product has abuse-deterrent properties does not mean that there is no risk of abuse. It means, rather, that the risk of abuse is lower than it would be without such properties. Because opioid products must in the end be able to deliver the opioid to the patient, there may always be some abuse of these products.

In the FDA Guidance, abuse-deterrent properties are defined as those properties shown to meaningfully deter abuse, even if they do not fully prevent abuse. The term abuse is defined as the intentional, non-therapeutic use of a drug product or substance, even once, to achieve a desirable psychological or physiological effect. Abuse is not the same as misuse, which refers to the intentional therapeutic use of a drug product in an inappropriate way and specifically excludes the definition of abuse. The FDA Guidance uses the term "abuse-deterrent" rather than "tamper-resistant" because the latter term refers to, or is used in connection with, packaging requirements applicable to certain classes of drugs, devices, and cosmetics.

The science of abuse deterrence is relatively new, and both the formulation technologies and the analytical, clinical, and statistical methods for evaluating those technologies are rapidly evolving. The FDA Guidance identifies seven categories of abuse-deterrent formulations:
1. Physical/chemical barriers - Physical barriers can prevent chewing, crushing, cutting, grating, or grinding of the dosage form. Chemical barriers, such as gelling agents, can resist extraction of the opioid using common solvents like water, simulated biological media, alcohol, or other organic solvents. Physical and chemical barriers can limit drug release following mechanical manipulation, or change the physical form of a drug, rendering it less amenable to abuse.
2. Agonist/antagonist combinations - An opioid antagonist can be added to interfere with, reduce, or defeat the euphoria associated with abuse. The antagonist can be sequestered and released only upon manipulation of the product. For example, a drug product can be formulated such that the substance that acts as an antagonist is not clinically active when the product is swallowed, but becomes active if the product is crushed and injected or snorted.
3. Aversion - Substances can be added to the product to produce an unpleasant effect if the dosage form is manipulated or is used at a higher dosage than directed. For example, the formulation can include a substance irritating to the nasal mucosa if ground and snorted.
4. Delivery System (including use of depot injectable formulations and implants) - Certain drug release designs or the method of drug delivery can offer resistance to abuse. For example, sustained-release depot injectable formulation or a subcutaneous implant may be difficult to manipulate.
5. New molecular entities and prodrugs- The properties of a new molecular entity (NME) or prodrug could include the need for enzymatic activation, different receptor binding profiles, slower penetration into the central nervous system, or other novel effects. Prodrugs with abuse-deterrent properties could provide a chemical barrier to the in vitro conversion to the parent opioid, which may deter the abuse of the parent opioid. New molecular entities and prodrugs are subject to evaluation of abuse potential for purposes of the Controlled Substances Act (CSA).
6. Combination - Two or more of the above methods could be combined to deter abuse.
7. Novel approaches - This category encompasses novel approaches or technologies that are not captured in the previous categories.

Although there are already a number of proposals available based on the categories 1 to 5, above, there is still an unmet need to provide efficient combinations of these methods and approaches and, especially, to provide novel approaches.

It is therefore the object of the present invention to provide a new approach and technology for abuse-deterrent drugs, especially for abuse-deterrent opioid formulations.

Therefore, the present invention provides a pharmaceutical composition comprising a drug with a laccase-reactive functional group and a laccase (EC 1.10.3.2).

Laccases (EC 1.10.3.2; CAS registry number 80498-15-3) are frequently described and analysed copper-containing oxidase enzymes that are found in many plants, fungi, and microorganisms (Riva, Trends in Biotechnology 24 (2006), 219-226). Laccases act on phenols and similar molecules, performing one-electron oxidations. According to the BRENDA database, the systematic name of laccase is benzenediol:oxygen oxidoreductase. Laccases are defined as a group of multi-copper proteins of low specificity acting on both o- and p-quinols, and often acting also on aminophenols and phenylenediamine. The semiquinone may react further either enzymatically or non-enzymatically.

Additionally, some non-laccase substrates can be oxidised by using mediators for the reaction. When these well-established laccase mediators are oxidized by laccase they generate strong oxidizing reactive species (radicals, radical quinones and quinones) which can further react with non-laccase substrates. If the laccase oxidized mediator is reduced back to its original compound, it is again re-oxidized by laccase to generate reactive species which can again oxidize another molecule. Such laccase mediators are able to oxidize molecules which cannot be oxidized directly by laccase. There are many types of mediators used in laccase formulations including aromatic centered mediators (ArOH; e.g. ferulic acid, syringaldehyde) and nitrogen centered mediators (RNOH; e.g. violuric acid, 1-hydroxybenzotriazole.

Another strategy involves the use of co-substrates together with laccase. Co-substrates are added to the reaction and they are oxidized by the laccase subsequently reacting and forming covalent bonds with non-laccase substrates resulting in oligomers or polymers. Generally many laccase substrates can act as cosubstrates to bind to non-laccases substrates such as phenolics (catechol), aromatic amines, alkenes, etc..

Laccases play a role in the formation of lignin by promoting the oxidative coupling of monolignols, a family of naturally occurring phenols. Laccases can be polymeric, and the enzymatically active form can be a dimer or trimer. Other laccases, such as the ones produced by the fungus Pleurotus ostreatus, play a role in the degradation of lignin, and can therefore be included in the broad category of lignin-modifying enzymes. Because laccase belongs to the oxidase enzyme family it requires oxygen as a second substrate for the enzymatic action. Spectrophotometry can be used to detect laccases, using the substrates ABTS (2,2'-azino-bis-(3-ethylbenzthiazoline-6-sulfonic acid), syringaldazine, 2,6-dimethoxyphenol, and dimethyl-p-phenylenediamine. Activity can also be monitored with an oxygen sensor, as the oxidation of the substrate is paired with the reduction of oxygen to water.

The present invention provides laccase as a novel approach for abuse-deterrent pharmaceutical drug formulations, especially opioid formulations. The new strategy of the present invention is based on the use of laccase (or an enzyme system comprising laccase) which convert the drug into a non-active form (e.g. into a precipitated or inactivated product) making it non-useable (e.g. by transforming it into an inactive (or at least: less active) form or by making it impossible to inject the drug with a syringe (in vitro)). On the other hand, if the drug is administrated as foreseen (e.g. orally), proteases from the body deactivate the laccase and the drug can unfold its effect without being inactivated by the accompanying laccase. For an improved protease-degradability, the laccase may even be modified by the introduction of (additional) protease cleavage sites so as to increase the degradation rate of the laccase after exposure to such proteases.

A prerequisite of the abuse-deterrent strategy according to the present invention is that the drug has a functional group which reacts with laccase. A further prerequisite is that during storage and before use the composition according to the present invention containing a combination of the drug and laccase is kept in an environment wherein laccase does not (yet) react with the drug. This can e.g. be safeguarded by keeping (storing) both components under conditions where laccase is inactive or by spatial separation of the two components. For example, if the composition is kept in a dry form, laccase cannot react with the drug because such reaction requires aqueous conditions. As soon as a dry composition according to the present invention is dissolved in water (e.g. in order to extract the opioid drug for abuse), laccase can react with the drug and enzymatically transforms the drug into a molecule that is not (ab-)usable anymore because of its degradation or because it is polymerised. Another example for preventing the laccase to react on the drug is to spatially separate laccase from the drug so that - again - only after contact of the composition with water or a comparable solvent, laccase can react on the drug. Such spatial separation can e.g. be established by providing laccase and the drug in different compartments of the pharmaceutical formulation, by providing specific coatings, by separate granulation, etc..

On the other hand, the laccase in the compositions according to the present invention must be reactive, e.g. once exposed to aqueous environment or to other situations which are not in line with the administration routes or administration activities intended (i.e. if an abuse is likely), it has to react with the accompanied drug to prevent or at least deter abuse of this drug.

The drug contained in the composition according to the present invention has therefore to contain a functional group that can be accessed and modified by the laccase activity. Accordingly, the pharmaceutical composition according to the present invention comprises a drug, wherein the laccase-reactive functional group is a phenolic hydroxyl-group, furthermore the following organic substances can be oxidized by laccases: aminophenols, benzenethiols, di- or polyphenols, methoxy-substituted phenols, amino phenols, diamines, aromatic amines, polyamines, ascorbate, hydroxyindoles, aryl diamines, anilins, preferably substances with a phenolic hydroxyl-group (Morozova et al., Biochemistry (Moscow) 72 (2007), 1136-1150; Madhavi et al., Bioresources 4 (2009), 1694-1717; Alcalde, Industrial Enzymes, Springer Verlag, Chapter 26; Jeon et al., Trends in Biotechnology 31 (2013), 335-341). Accordingly, the drugs used according to the present invention are opioids comprising such groups and/or drugs belonging to the substance classes of benzenethiols, mono-, di- and polyphenols, methoxy-substituted phenols, aminophenols, diamines, aromatic amines, polyamines, ascorbates, hydroxyindols, aryl diamines, and anilins.

It is also possible to use reaction mediators in the composition according to the present invention which mediate the laccase reaction with the drug.

Mediators are small-molecular weight compounds that act as electron shuttle systems and broaden the substrate specificity of laccases towards more recalcitrant compounds. Often these substances cannot enter the active site of the enzyme or their redox-potential is too high. It has been shown, that aromatic contaminants like dyes, polycyclic aromatic hydrocarbons and chlorophenols were successfully detoxified as well as various organophosphorus compounds and sulfonamide antibiotics were successfully degraded using a laccase in combination with a mediator (Morozova et al., Applied Biochemistry and Microbiology, 43/5 (2007), 523-535; Canas et al., Biotechnology Advances, 28 (2010), 694-705; Johannes et al., Applied and Environmental Microbiology, 66/2 (2000), 524-528; Trovaslet-Leroy et al., Chemico-Biological Interactions, 187/1-3 (2010), 393-396; Wenig et al., Bioresource Technology, 141 (2013), 152-159).

The laccase used in the present composition can be selected easily and optimised by availability, reactivity with the selected drug and the planned storage and administration conditions. Laccases are available from various sources, e.g. from fungi, bacteria or plants. Most of the common laccases can also be produced recombinantly. Recombinant production is preferred if consistent and continuous amounts are needed and in cases where natural sources are problematic, e.g. due to possible impurities. There are a number of laccases that are industrially used and therefore well available also in amounts necessary for mass production. For example fungal laccases are available from Collybia, Fomus, Lentinus, Pleurotus, Aspergillus, Neurospora, Podospora, Phlebia (P. radiata), Coriolus (C. hirsitus), Botrytis, Polyporus (P. pinsitus or P. versicolor, Rhizoctonia solani, Scytalidium (S. thermophilium), Pyricularia (P. oryzae), Coprinus (C. cinereus), Trametes, (T. hirsuta, T. villosa and T. versicolor), Coriolopsis (C. gallica), Phanerochaete chrysosporium, Heterobasidion annosum, Spiniger meineckellus and Myceliophthora thermophila; bacterial laccases are available from Bacillus, Pseudomonas, Streptomyces and Azospirillum. Specifically preferred laccases are from Trametes villosa, from Myceliophthora thermophila, and from Pleurotus ostreatus.

The drug which is usable with the present invention is not critical, as long as it contains a functional group that is modifiable by the laccase activity. Of course, preferred drug to be subjected to the present new approach for abuse-deterrent products are the drugs with known abuse risk. Accordingly, preferred drugs contained in the pharmaceutical compositions according to the present invention are opioid drugs with a laccase-reactive functional group.

Opioids are substances that act on the nervous system in a similar way to opiates such as morphine and codeine. Opiates, i.e. analgesic alkaloid compounds found naturally in the opium poppy plant Papaver somniferum, therefore form a sub-group of opioids. Opioids may therefore be extracted from opium or made artificially. In the medical context, opioids are primarily used for the treatment of pain. The significant side effects of opioids, such as sedation, and a strong sense of euphoria, also expose these drugs to misuse and abuse. Opioid dependence can develop with ongoing administration, leading to a withdrawal syndrome with abrupt discontinuation. For example, morphine-like opioids are well known for their addictive properties, and for their ability to produce euphoria, motivating some to use opioids recreationally. They can cause death in overdose from respiratory depression. In 2013 between 28 and 38 million people used opioids recreationally (0.6% to 0.8% of the global population between the ages of 15 and 65; World Drug Report 2015).

Specifically preferred opioids used as drugs in the compositions according to the present invention are therefore opioids comprising a phenolic hydroxy-group, such as morphine, tapentadol, hydromorphone, etorphine, desomorphine, oxymorphone, buprenorphine, opioid peptides comprising a phenylalanine residue, such as adrenorphin, amidorphin, casomorphin, DADLE ([D-Ala², D-Leu⁵]-Enkephalin), DAMGO ([D-Ala², N-MePhe⁴, Gly-ol]-enkephalin), dermorphin, endomorphin, morphiceptin, and TRIMU 5 (L-tyrosyl-N-{[(3-methylbutyl)amino]acetyl}-D-alaninamide); oripavine, 6-MDDM (6-methylenedihydrodesoxymorphine), chlornaltrexamine, dihydromorphine, hydromorphinol, methyldesorphine, N-phenethylnormorphine, RAM-378 (7,8-Dihydro-14-hydroxy-N-phenethylnormorphine), heterocodeine, dihydroheterocodeine, 7-spiroindanyloxymorphone, morphinone, pentamorphone, semorphone, chloromorphide, nalbuphine, oxymorphazone, 1-iodomorphine, morphine-6-glucuronide, 6-monoacetylmorphine, normorphine, morphine-N-oxide, cyclorphan, dextrallorphan, levorphanol, levophenacylmorphan, norlevorphanol, oxilorphan, phenomorphan, furethylnorlevorphanol, xorphanol, butorphanol, 6,14-endoethenotetrahydrooripavine, BU-48 (N-Cyclopropylmethyl-[7α,8α,2',3']-cyclohexano-1'[S]-hydroxy-6,14-endo-ethenotetrahydronororipavine), buprenorphine, cyprenorphine, dihydroetorphine, norbuprenorphine, 5'-guanidinonaltrindole, diprenorphine, levallorphan, meptazinol, methylnaltrexone, nalfurafine, nalmefene, naloxazone, naloxone, nalorphine, naltrexone, naltriben, naltrindole, 6β-naltrexol-d4, pseudomorphine, naloxonazine, norbinaltorphimine, alazocine, bremazocine, dezocine, ketazocine, metazocine, pentazocine, phenazocine, cyclazocine, hydroxypethidine (bemidone), ketobemidone, methylketobemidone, propylketobemidone, alvimopan, picenadol and pharmaceutically acceptable salts, hydrates, solvates, esters, prodrugs and mixtures thereof, preferably morphine, tapentadol, oxymorphone, hydromorphone, buprenorphine, especially morphine. An example of an opioid drug with a phenolic amino group is anileridine.

The present pharmaceutical composition can be formulated according to the intended (non-abuse-deterrent) administration route, if necessary, adapted to the present invention (e.g. when laccase and the drug are spatially separated). Preferably, the pharmaceutical composition according to the present invention is provided as a dose unit. It is therefore preferred to provide the present composition as a tablet, a coat-core tablet, a bi-layer tablet, a multi-layer tablet, a sublingual and a buccal tablet, a sublingual film, a capsule, a pellet, a MUPS (multiple unit pellet system), a granulate, a powder, especially coated, sugar-coated and/or functionally coated (e.g. enteric coated) forms thereof. Enteric coatings are specifically advantageous for the prevention of premature inactivation of laccase, i.e. before the physiologic proteases act on the laccase. An enteric coating is generally defined as a polymer barrier applied on oral medication for protecting drugs from the acidity of the stomach. Enteric coatings are therefore usually stable at the highly acidic pH found in the stomach, but break down rapidly at a less acidic (relatively more basic; pH 7-9) pH in the intestine (i.e. after leaving the stomach). Typical substances/materials used for such enteric coatings are shellac, cellulose acetate phthalate (CAP), polyvinyl acetate phthalate (PVAP), hydroxypropylmethylcellulose phthalate, and methacrylic acid ester copolymers, or zein, for example, as well as hydrophobic or hydrophilic polymers and mixtures of such substances, if appropriate. Hydrophobic polymeric coatings include acrylic polymer, acrylic copolymer, methacrylic polymer or methacrylic copolymer, including Eudragit^{®} L100, Eudragit^{®} L100-55, Eudragit^{®} L 30 D-55, Eudragit^{®} S100, Eudragit^{®} 4135F, Eudragit^{®} RS, acrylic acid and methacrylic acid copolymers, methyl methacrylate, methyl methacrylate copolymers, ethoxyethyl methacrylates, cyanoethyl methacrylate, aminoalkyl methacrylate copolymer, polyacrylic acid, polymethacrylic acid, methacrylic acid alkylamine copolymer, polymethyl methacrylate, polymethacrylic acid anhydride, polymethacrylate, polyacrylamide, polymethacrylic acid anhydride and glycidyl methacrylate copolymers, an alkylcellulose such as ethylcellulose, methylcellulose, carboxymethyl cellulose, hydroxyalkylcellulose, hydroxypropyl methylcelluloses such as hydroxypropyl methylcellulose phthalate, and hydroxypropyl methylcellulose acetate succinate, cellulose acetate butyrate, cellulose acetate phthalate, and cellulose acetate trimaleate, polyvinyl acetate phthalate, polyester, waxes, shellac, zein, or the like. The coating can also include hydrophilic materials such as a pharmaceutically-acceptable, water-soluble polymer such as polyethylene oxide (PEO), ethylene oxide-propylene oxide co-polymers, polyethylene-polypropylene glycol (e.g. poloxamer), carbomer, polycarbophil, chitosan, polyvinyl pyrrolidone (PVP), polyvinyl alcohol (PVA), hydroxyalkyl celluloses such as hydroxypropyl cellulose (HPC), hydroxyethyl cellulose, hydroxymethyl cellulose and hydroxypropyl methylcellulose, carboxymethyl cellulose, sodium carboxymethyl cellulose, methylcellulose, hydroxyethyl methylcellulose, hydroxypropyl methylcellulose, polyacrylates such as carbomer, polyacrylamides, polymethacrylamides, polyphosphazines, polyoxazolidines, polyhydroxyalkylcarboxylic acids, alginic acid and its derivatives such as carrageenate alginates, ammonium alginate and sodium alginate, starch and starch derivatives, polysaccharides, carboxypolymethylene, polyethylene glycol, natural gums such as gum guar, gum acacia, gum tragacanth, karaya gum and gum xanthan, povidone, gelatin or the like.

Preferred enteric coating materials are methyl acrylate-methacrylic acid copolymers, cellulose acetate succinate, hydroxy propyl methyl cellulose phthalate, hydroxy propyl methyl cellulose acetate succinate, polyvinyl acetate phthalate (PVAP), methyl methacrylate-methacrylic acid copolymers, shellac, cellulose acetate trimellitate, sodium alginate and zein.

A further embodiment of the pharmaceutical composition according to the present invention is a coated composition wherein the coat comprises the laccase. The laccase acts on the drug when the coated composition is dissolved in aqueous liquids (for extracting the drugs (opioids)), whereas the laccase is immediately destroyed or inactivated after ingestion (according to the prescribed (oral) administration route) in the stomach or by the proteases in the intestine.

A specifically preferred embodiment is a composition wherein laccase is coated so as to keep the laccase inactive on the drug as long as the planned administration route is followed, i.e. which keeps the laccase activity from the drug while being coated, e.g. until excretion of the (still coated) laccase by the patient, whereas abuse activity destroys the coat so that the laccase immediately acts on the drug. For example, the laccase may be coated by one or more of the aforementioned substances/materials used for enteric coatings. Specifically preferred examples of such film-coatings or sequestering materials are Eudragit^{®} RS (30D), Eudragit^{®} NE 30 D; ethylcellulose, polymethacrylates (all disclosed in Rowe et al. (Eds.) "Handbook of Pharmaceutical Excipients" 6th Ed. (2009), Pharmaceutical Press, pages 262ff., 525 ff.); or the substances used in other opioid compositions for coating opioid antagonists (see e.g. EP 2 034 975 B1, US 8,465,774 B2, and WO 2004/026283 A1), except, of course and self-understanding, substances that have a degradation risk by laccase activity (e.g. because they contain a group that is converted by the laccase provided in the composition according to the present invention).

The pharmaceutical composition preferably contains the drug in the amount foreseen in the non-abuse-deterrent original composition. Accordingly, the drug is preferably contained in an amount of 0.1 to 5.000 mg, preferably 0.5 to 1.000 mg, especially 1 to 500 mg, per dosage unit. Depending on the formulation and the expected laccase activity during normal administration, also an increase of the drug amount may be provided (compared to the non-abuse-deterrent original composition, i.e. to the pharmaceutical formulation of the drug without the abuse-deterrent features) e.g. to compensate possible loss of drug activity (if such minor loss of activity is likely or cannot be completely excluded).

The amount of laccase in the present pharmaceutical composition can be adjusted mainly based on the amount of drug, the source of laccase and the formulation details. According to a preferred embodiment, the laccase is contained in an amount of 1 to 1.000 units, preferably 10 to 100 units. There are several ways to determine laccase activity. For the present invention, activities are determined by monitoring the oxidation of ABTS spectrophotometrically at 420 nm (ε= 11.4 mL µmol⁻¹ cm⁻¹) at 25°C in 100 mM sodium phosphate buffer (pH 7). The enzyme activity is expressed in Units, whereas one Unit (U) is defined as the amount of enzyme that catalyses the conversion of 1 µmole of ABTS per minute (1 U = µmol min⁻¹) (hereinafter referred to as the "laccase test according to the present invention").

Although the present invention provides a completely new strategy for abuse-deterrent drugs, the present novel approach is also combinable with other abuse-deterrent strategies, e.g. the ones that have been identified in the FDA Guidance 2015 or in Schaeffer, J. Med. Toxicol. 8 (2012), 400-407. Accordingly, the pharmaceutical composition according to the present invention preferably comprises a further abuse-deterrent feature, for example a feature selected from the group consisting of: a physical or chemical barrier, especially increased tablet hardness, a (laccase-insensitive) drug antagonist, an aversion component, an abuse-deterrent delivery system and a prodrug. Provision of a physical barrier, especially increased tablet hardness, or an aversion component, especially a gelling agent and/or a non-gelling viscosity-increasing agent (e.g. λ-carrageenan) or, e.g. an emetic or a nasal irritant, is specifically preferred. For example, the present composition can be provided as a formulation with a resistance of more than 400 N, especially more than 500 N, as prescribed in the European Pharmacopeia (Ph.Eur.8 (2014) 2.9.8). Further examples for abuse-deterrent features combinable with the present invention are the provision of discrete mechanically reinforcing particles (WO 2012/061779 A1), of materials that are both hydrophilic and hydrophobic (WO 2012/112952 A1), of an acid soluble ingredient (a cationic polymer) with a buffering ingredient (US 9,101,636 B2), of a monolithic solidified oral dosage form prepared by a thermal process (US 2008/0075771 A1), of an emetic or a nasal irritant (US 2008/075771 A1), of an extruded formulation (US 2015/0057304 A1) or of amorphous or polymeric organic acid salts of the opioid (US 2015/016835 A1), etc..

According to a preferred embodiment the pharmaceutical composition comprises a matrix containing 1 to 80 wt.% of one or more hydrophobic or hydrophilic polymers, preferably a matrix comprising agar, alamic acid, alginic acid, carmellose, carboxymethylcellulose sodium, carbomer (such as Carbopol^{®} carrageenan, chitosan , especially carboxymethylchitosan, catechol, copovidone, dextrin, gelatin, guar gum, hydroxyethyl cellulose, hydroxypropyl cellulose, hydroxypropyl methylcellulose, methacrylic acid copolymer, methylcellulose derivatives, microcrystalline cellulose, polyacrylic acid, polyalkylene oxide, especially polyethylene glycol, polyvinyl alcohol, polyvinyl acetate, povidone, propylene glycol alginate, a polyvinylcaprolactam-polyvinyl acetate-polyethylene glycol graft co-polymer, pullulan, silicon dioxide, sodium alginate, starch, vinylpyrrolidone-vinyl acetate copolymers, xanthan gum; or of non-polymer matrix formers like microcrystalline wax, fatty alcohols and fatty acids like stearyl alcohol, cetyl stearyl alcohol, stearic acid, palmitic acid or salts and mixtures thereof, mono-, di- and triglycerides of saturated fatty acids with a chain length between 16 and 22 carbon atoms and a mixture of such mono- di- and triglycerides, respectively.

According to a preferred embodiment of the present invention, the pharmaceutical composition comprises a hydrogel-forming component and/or suitable crosslinkers which allows the generation of insoluble crosslinked hydrogels of the drug once laccase is activated by abusive steps. Preferred hydrogel-forming components are chitosan and carboxymethylchitosan; preferred crosslinkers are phenolic crosslinkers, especially catechol and vanillin. Preferred examples of such hydrogel/crosslinker compositions are composition comprising chitosan and catechol or compositions comprising carboxymethylchitosan and vanillin.

The pharmaceutical composition according to the present invention is preferably a storage stable composition, preferably by comprising less than 5%, especially less than 1%, laccase-processed drug after 6 month storage at 25°C under dry conditions (laccase activity being determined by the laccase test according to the present invention).

In general, laccases are preferred that are acid-labile so that the laccase activity of the present composition is immediately inactivated as soon as the laccase is in contact with the stomach fluid and/or the intestine environment of the patient to whom the composition is administered.

The present pharmaceutical composition can be provided as a modified release composition, especially a prolonged release composition. The term "modified form" is meant to include accelerated release, controlled release, and sustained release forms. Certain release forms can be characterized by their dissolution profile. "Dissolution profile", means a plot of amount of active ingredient released as a function of time (Ph.Eur.8 (2014) 2.9.3). The dissolution profile can be measured utilizing the Drug Release Test <724>, which incorporates standard test USP 26 (Test <711>) of the US Pharmacopeia. A profile is characterized by the test conditions selected. Thus, the dissolution profile can be generated at a preselected apparatus, shaft speed, temperature, volume, and pH of the dissolution media. A first dissolution profile can be measured at a pH level approximating that of the stomach. A second dissolution profile can be measured at a pH level approximating that of one point in the intestine or several pH levels approximating multiple points in the intestine. A highly acidic pH may simulate the stomach and a less acidic to basic pH can simulate the intestine. By the term "highly acidic pH" it is meant a pH of about 1 to about 4. By the term "less acidic to basic pH" is meant a pH of greater than about 4 to about 7.5, preferably about 6 to about 7.5. A pH of about 1.2 can be used to simulate the pH of the stomach. A pH of about 6.0 to about 7.5, preferably about 7.5 can be used to simulate the pH of the intestine.

In contrast to modified release, "immediate release" is the conventional or non-modified release form in which greater than or equal to about 50% or more preferably about 75% of a drug according to the present invention is released e.g. within two hours of administration, preferably within one hour of administration, especially within 30 min of administration. By "controlled release" a dosage form is meant in which the drug release is controlled or modified over a period of time. Controlled can mean, for example, accelerated, sustained, delayed or pulsed release at a particular time. Alternatively, controlled can mean that the drug release is extended for longer than it would be in an immediate release dosage for, i.e., at least over several hours.

"Delayed release" indicates that there is a time-delay before significant plasma levels of the drug are achieved. A delayed release formulation according to the present invention avoids an initial burst of the drug, or can be formulated so that drug release in the stomach is avoided. A "pulsed release" formulation can contain a combination of immediate release, sustained release, and/or delayed release formulations in the same dosage form. A "semi-delayed release" formulation is a "pulsed released formulation in which a moderate dosage is provided immediately after administration and a larger dosage some hours after administration.

The terms "sustained release" or "extended release" are meant to include the release of the drug at such a rate that blood (e.g., plasma) levels are maintained within a therapeutic range but below toxic levels for at least about 8 hours, preferably at least about 12 hours, especially at least 24 hours (specifically preferred for 1 per day dosage regimen) after administration at steady-state. The term "steady-state" means that a plasma level for a given drug has been achieved and which is maintained with subsequent doses of the drug at a level which is at or above the minimum effective therapeutic level and is below the minimum toxic or overdose plasma level for a given drug. Most of the polymeric matrices and non-polymer matrix formers mentioned above have the capacity to deliver prolonged or sustained release capacity for a given drug in the formulation according to the present invention.

Specific examples for morphine retard matrices based on acrylic acid polymers are disclosed in EP 0 682 945 B1; a tapentadol retard matrix based on HPMC is disclosed in EP 1 439 829 B1.

According to a preferred embodiment, the pharmaceutical composition according to the present invention comprises the target drug alone (as the only effective ingredient) or in combination with other pharmaceutically active ingredients, preferably in combination with a non-opioid analgesic, especially with ibuprofen, diclofenac, naproxen, paracetamol and acetyl-salicylic acid.

In a preferred embodiment, the enzyme in the pharmaceutical composition essentially does not act on the drug in vivo when the composition is swallowed intact.

Preferably, the enzyme in the pharmaceutical composition according to the present invention is present in the composition in an essentially non-releasable form when the composition is swallowed intact.

According to a preferred embodiment of the present invention the laccase enzyme in the present pharmaceutical composition is deactivated in vivo.

According to a further aspect, the present invention also relates to a method for manufacturing a pharmaceutical composition according to the present invention comprising the steps of mixing the drug with the laccase and finishing the mixture to a pharmaceutical composition. Alternatively, the method for manufacturing the pharmaceutical composition according to the present invention comprises the steps of providing the drug and the laccase in separated form and finishing the separated drug and laccase to a pharmaceutical composition.

According to another aspect, the present invention provides a composition comprising an opioid drug and a hydrogel-forming component and/or a crosslinker. The hydrogel-forming component and/or the crosslinker allow the generation of insoluble crosslinked molecules, especially as hydrogel, of the drug once the pharmaceutical composition is mixed with an aqueous mixture in the process of abusive steps. Preferred hydrogel-forming components are chitosan and carboxymethylchitosan; preferred crosslinkers are phenolic crosslinkers, especially catechol and vanillin. Preferred examples of such hydrogel/crosslinker compositions are composition comprising chitosan and catechol or compositions comprising carboxymethylchitosan and vanillin.

Preferably, the compositions according to the present inventions are provided as hydrogels. This involves the inclusion of another abuse deterrent approach, namely to create a system which changes the rheological properties of the reaction solution, i.e. the formation of a hydrogel. Hydrogels are very viscous and as a consequence, the drawing up with a needle is not possible.

The present invention is further illustrated by the following examples and the figures, yet without being restricted thereto.

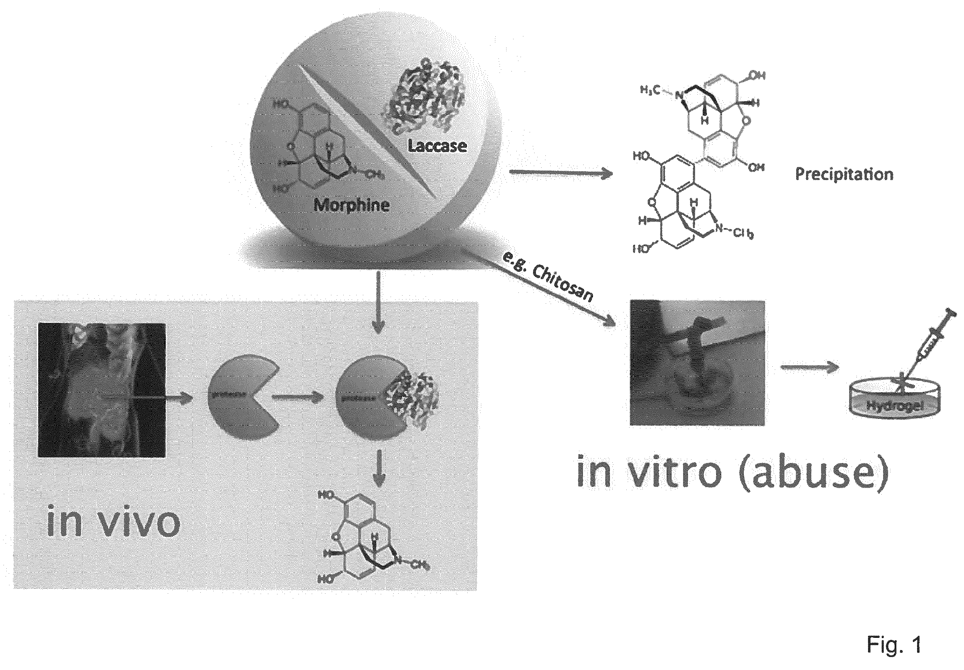

Fig. 1 shows the strategy of the present invention, exemplified by the example of morphine as drug. The abuse deterrent morphine/laccase system according to the present invention either converts morphine into a precipitated product or creates in combination with additives a hydrogel with which it is not possible anymore to inject the drug with a syringe. If the drug is administrated as foreseen, proteases from the body deactivate the laccase and the drug unfolds its effect.
Fig. 2 shows the opioids that are investigated in the examples (morphine (left), tapentadol (middle) and oxycodone (right)).
Fig. 3 shows laccase oxidation of morphine with Myceliophthora thermophila (MtL) (violet) and Trametes villosa (TvL) (green).
Fig. 4 shows laccase oxidation of tapentadol with MtL (violet) and TvL (green).
Fig. 5 shows laccase oxidation of oxycodone with MtL (green) and TvL (violet).
Fig. 6 shows a TLC of morphine after enzymatic conversion at different time points compared to morphine as reference (first lane).
Fig. 7 shows a TLC of tapentadol after enzymatic conversion at different time points compared to tapentadol as reference (first lane).
Fig. 8 shows an FTIR of morphine precipitate (red line: morphine reference, blue line: precipitated morphine polymer)
Fig. 9 shows HPLC measurement of the enzymatically oxidized morphine.
Fig. 10 shows GC-MS spectra of morphine after enzymatic conversion.
Fig. 11 shows GC-MS spectra of morphine out of a GC-MS database library; comparison of the measured spectrum to the database spectrum.
Fig. 12 shows the conversion of morphine by laccase, detected by GC-MS.
Fig. 13 shows the chitosan-catechol-morphine hydrogel.
Fig. 14 shows the carboxymethylchitosan-morphine hydrogel.

### Example:

### Laccase for use in abuse-deterrent opioid formulations

Opioids are an important component of modern pain management, though the abuse and/or misuse of those drugs has created a growing public health problem. To counteract this problem, there is a strong demand for new technologies for preventing the abuse of opioids. This project therefore tries to approach this problem with a very unique way based on using enzymes.

The potential of enzymes to polymerize opioids, thereby preventing abuse were investigated. These enzymes will not be active when opioids are administrated correctly. These possibilities are thoroughly investigated by the present project.

In the present example, the development of an appropriate enzyme system to eliminate opioids from solution is shown. Moreover, optimized reaction conditions for effective conversion of the opioid solution preventing administration through injection are provided. Finally, the function of the system is verified by showing the inactivation of the opioid destroying enzymes proteolytically when the drugs are administered as foreseen.

### 1. Materials

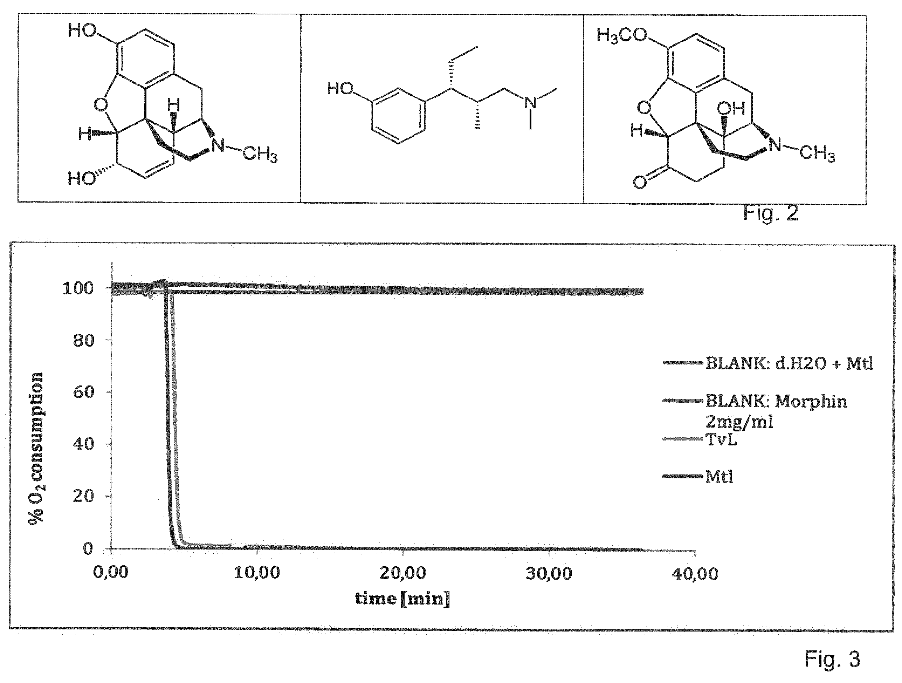

The opioids that are investigated were morphine, tapentadol and oxycodone as shown in Figure 2.

Two different laccases were used to achieve elimination of opioids - one of them originated from Trametes villosa (TvL), the other one from Myceliophthora thermophila (MtL).

### 2. Oxygen Measurements

To determine whether the laccases act on any of the given opioids (morphine, tapentadol, oxycodone), an optical oxygen sensor was used. Laccases use oxygen as electron acceptor, consequently the oxygen concentration decreases upon substrate oxidation.

The conditions for the following reactions are shown in Table 1:

**Table 1: Conditions for oxygen measurement of laccase catalysed oxidation of opioids**

| | |
|---|---|
| Enzyme (Laccase) | 10 U/ml |
| Substrate | 2 mg/ml |
| Solvent | Distilled water |
| Temperature | Room temperature |

In Figure 3 the oxidation of morphine by two different laccases namely from Myceliophthora thermophila (MtL) and Trametes villosa (TvL) is shown. The blue and red lines represent the two blanks, blue with distilled water and enzyme, and red distilled water with morphine. The violet and green lines represent the two reactions with the enzymes (violet: MtL, green: TvL). After approx. 5 minutes there is a clear decrease in the oxygen concentration in solution, meaning that the laccases are converting morphine to a reaction product. Additionally, the two solutions turned cloudy which means the reaction product precipitated. There was no significant difference between the two enzymes.

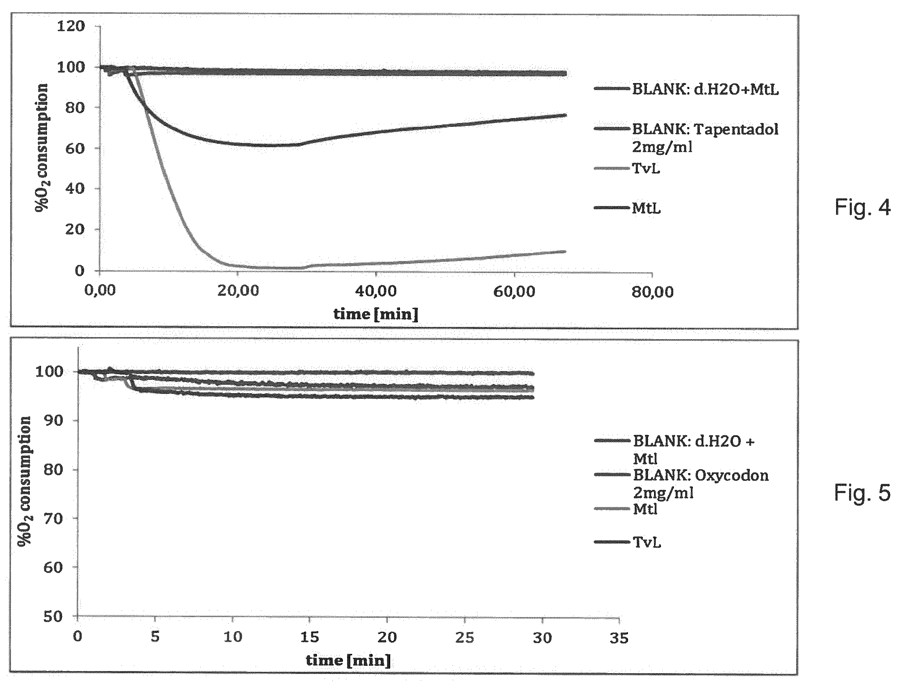

In Figure 4 the oxidation of tapentadol by MtL (violet) and TvL (green) is shown. The decrease of oxygen with MtL is much slower compared to the decrease of oxygen with TvL. This could be because TvL has a much higher redox potential than the MtL. The different curves also show that the specificity of the two enzymes is very different on tapentadol. TvL converts tapentadol better which is indicated by the slope of the curve as well as the fact that the curve in the case of TvL drops to almost 0% oxygen.

In Figure 5, the oxidation of oxycodone is shown. All of the curves look similar indicating that the two used enzymes are not capable of converting oxycodone. The cause for that is the missing phenolic - OH group in oxycodone (shown in Figure 2), which is needed by laccases.

### 3. Thin Layer Chromatography

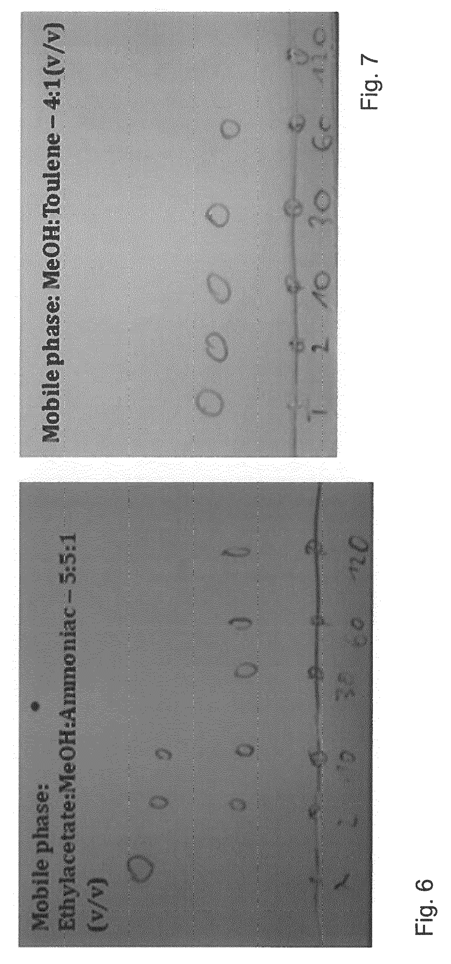

The reactions of morphine and tapentadol were also analysed by thin layer chromatography and are shown in Figure 6 and 7. Figure 6 shows the conversion of morphine. The first lane represents morphine as a reference, whereas the other lanes show samples that were taken at different time points (2, 10, 30, 60, 120 minutes). After only 2 minutes, 2 new dots are appearing on the TLC plate, indicating that a new product is formed out of the enzymatic reaction with morphine. The morphine reference dot disappears after 30 minutes, which indicates that all of the morphine is converted. The same procedure is shown in Figure 7 for tapentadol. The conversion of tapentadol is much slower, but there is also a new dot appearing after 2 minutes and the tapentadol dot is disappearing after 120 minutes.

### 4. Analysis of the precipitated product

As mentioned above the reaction product of morphine precipitated, consequently the next part was the analysis of this precipitate. The following conditions were used for the reaction (Table 2).

**Table 2: Conditions for the analysis of the morphine precipitate that is formed upon the enzymatic reaction with MtL**

| | |
|---|---|
| Enzyme | 20 U/ml |
| Substrate | 20 mg/ml |
| Solvent | Distilled water |
| Temperature | Room temperature |

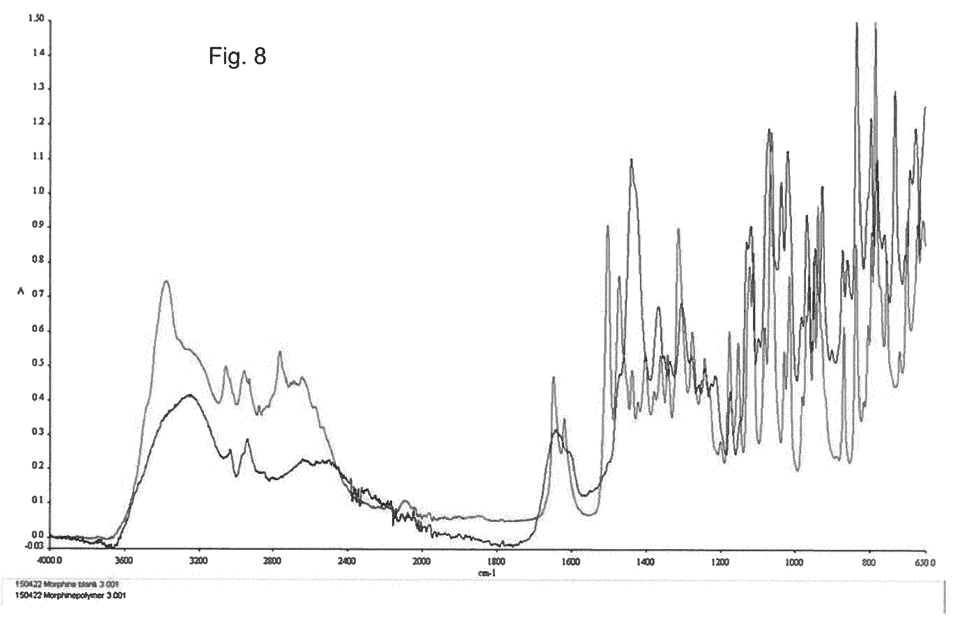

After 24 hours, the reaction mixture was centrifuged for 15 min at 16.100 rcf (relative centrifugal force). The supernatant was discarded and the remaining precipitate was lyophilized overnight. On the next day the dry precipitate was analyzed via FTIR as shown in Figure 8. The red line shows morphine, the blue line the precipitated reaction product. It can be seen that there are new peaks arising and other peaks decreasing due to the polymerization reaction of the morphine.

For further analysis a solubility test of the precipitate was performed. As indicated in Table 3, the results for the solubility test were ambivalent. In none of the used solvents the precipitate was soluble, most likely meaning that a high molecular weight product was formed upon the enzymatic reaction. This is desirable for the main goal of the project, but for further analysis a soluble compound is necessary.

**Table 3: Solubility tests of the precipitate formed upon the laccase-catalyzed oxidation of morphine**

| Solvent | Concentration of morphine precipitate [mg/ml] | Solubility |
|---|---|---|
| Acetonitrile | 0.4 | - |
| THF | 0.3 | - |
| Diethylether | 0.4 | - |
| Toluene | 0.4 | - |
| Hexane | 0.3 | - |
| 100 mM Citrate buffer pH4 | 1 | ~ |
| 100 mM Phosphate bufferpH3 | 1 | ~ |
| 50 mM Ammonium formate buffer pH3 | 0.001 | ~ |

### 5. Analysis of the precipitated product

To address the second task - the changing of the rheological properties of the reaction mixture - different additives were added to increase the viscosity of the solution. The tested additives are shown in Table 4 and were mixed to the reaction as stated. Most of the chosen additives are already used in pharmaceutical applications. The desired increase in viscosity was only achieved when using hydroxypropylmethylcellulose (HPMC).

**Table 4: Additives for the enzymatic morphine reaction to increase the viscosity**

| Additive | Ratio/concentration | Troubleshooting | Increased viscosity |
|---|---|---|---|
| Ferulic acid | 1:1 | | - |
| Catechol | 1:1 | toxic | - |
| Starch | 1:1 | solubility | - |
| Polyvinylpyrrolidone (PVP) | 50 mg/ml | solubility | - |
| Polyethylenglycol (PEG6000) | 50 mg/ml | | - |
| HPMC | 100 mg/ml | time | + |
| Catechin | 1:1 | | - |
| Neohesperidin dihydrochalcon | 1:1 | | - |

### 6. Measurements of enzymatic conversion using HPLC & GC

The kinetic of the enzymatic conversion of morphine was analyzed via HPLC and GC analysis.

Samples were taken at different time points (0, 2, 5, 10, 15, 30 minutes) during the reaction. The starting solution was 0.2 mg/ml morphine in distilled water. To start the reaction, MtL was added to a final concentration of 78.3 U/ml. 100µL sample were taken and put into 900 µL of methanol (MeOH) to precipitate the enzyme. The solution was centrifuged and the supernatant was transferred to an HPLC vial via a 0.2 µm filter. Then the solution was measured with following HPLC conditions shown in Table 5.

**Table 5: Conditions for the HPLC measurement of enzymatically oxidized morphine reaction rate determination**

| | |
|---|---|
| Column | Poroshell 120 EC-C18 3.0 x 0.5mm |
| Flow | 0.8 ml/min |
| Isocratic | 85% mQ H₂O, 5% MeOH, 10% formic acid |
| Injection volume | 5 µL |
| Column temperature | 40°C |
| Signal wave length | 240 nm |

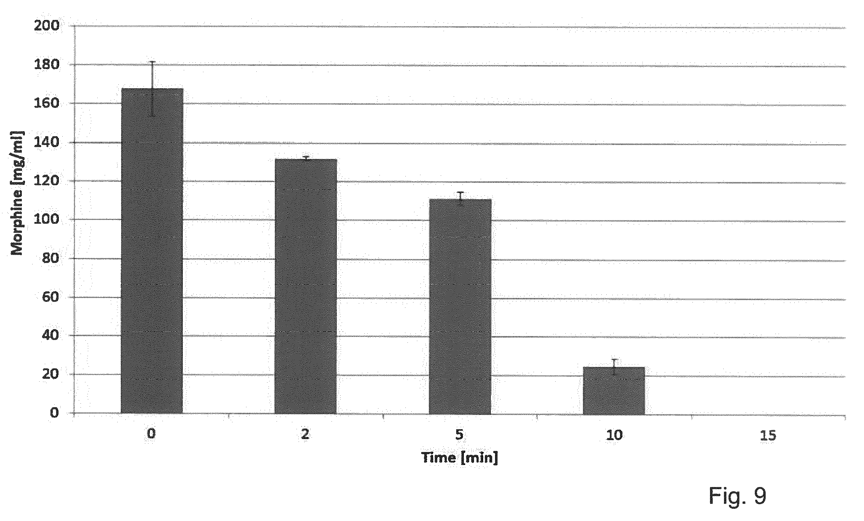

The result of the HPLC measurement is shown in Figure 9. It is clearly visible that morphine is converted by the laccase. After 15 minutes no morphine signal was measured anymore. This means after approx. 15 minutes 100% of the morphine was converted to a partly precipitated product. Further analysis of the exact reaction mechanism and reaction product has to be investigated.

In addition to the HPLC method, a GC-MS method was established and the samples were analysed using the following conditions shown in Table 6.

**Table 6: GC-MS conditions for enzymatically oxidized morphine reaction rate determination**

| Column | Agilent Technologies DB17MS |
|---|---|
| Temperature program | 120°C - 320°C |
| Run time | 11.5 min |

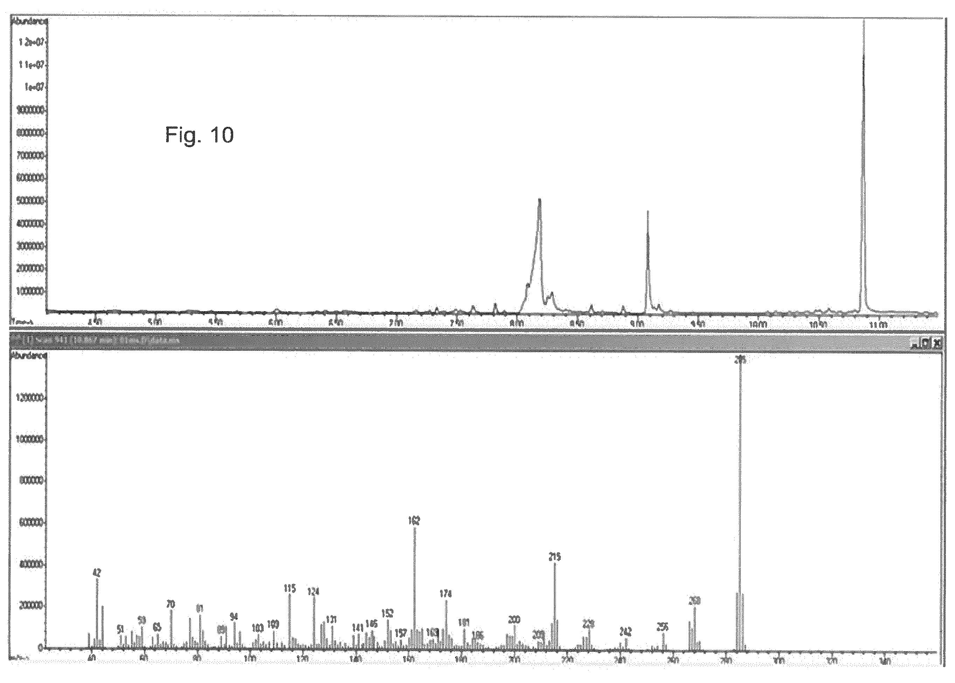

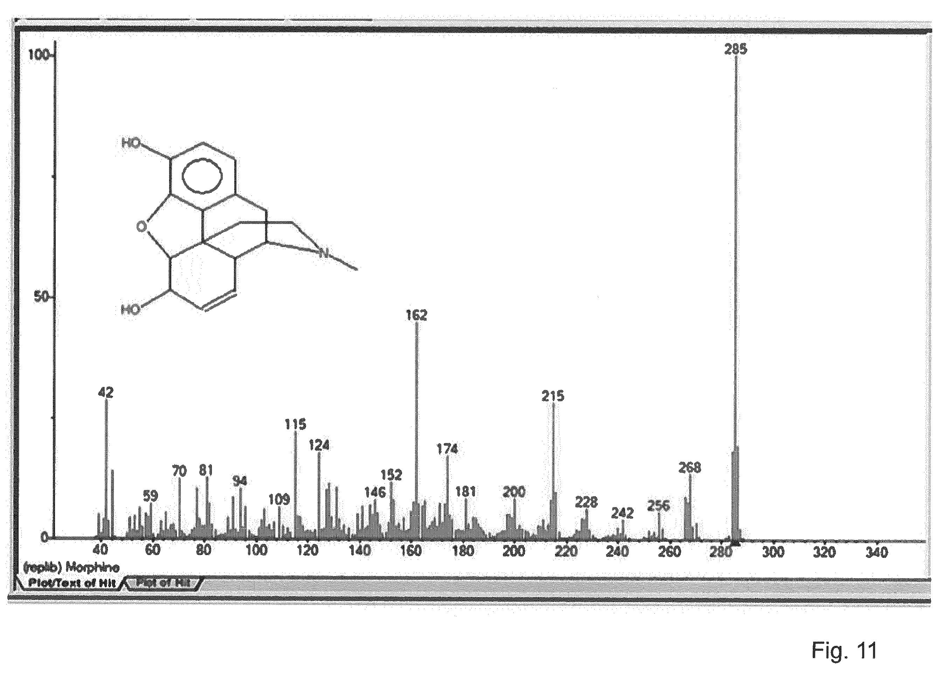

The MS spectrum in Figure 10 shows the result of the GC-MS measurement of morphine after the reaction. The sharp peak to the right represents morphine, which is confirmed by the MS spectrum library shown in figure 11. This is the proof that morphine was detected.

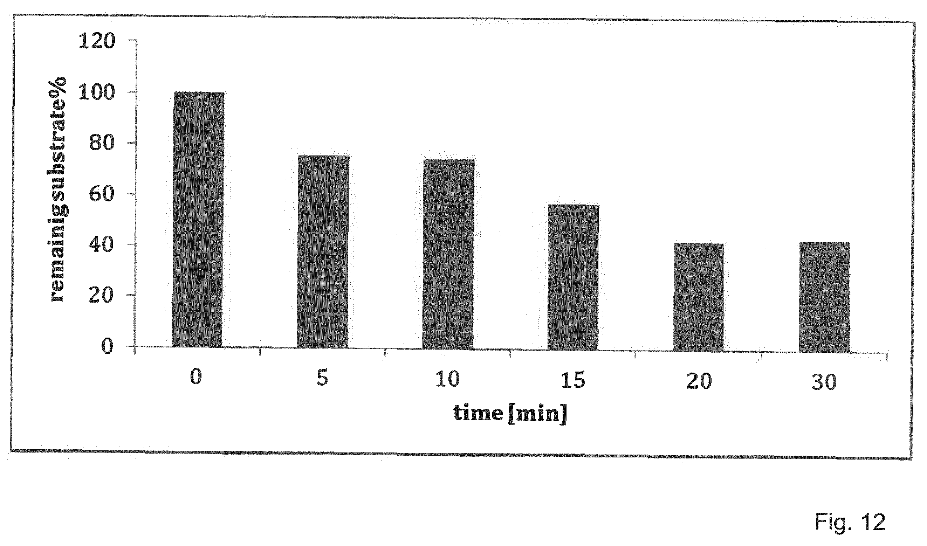

In Figure 12 the conversion rate of the reaction of morphine and laccase from *Myceliophthora thermophila* is shown. For this reaction a concentration of 2 mg/ml morphine in distilled water was used. To start the reaction MtL was added to a final concentration of 78.3 U/ml. For the sample preparation 100 µL sample were taken and added to 900 µL of methanol (MeOH) to precipitate the enzyme. The solution was centrifuged and the supernatant was transferred to an HPLC vial via a 0.2 µm filter. In the vial there was NaSO₄ to bind remaining water from the enzyme which could cause problems in the GC chromatograph.

The conversion rate, compared to the conditions of the ones mentioned above measured by HPLC, is much slower. After 30 minutes approx. 60% of the morphine is converted. The cause for this is most likely the different enzyme/substrate ratio. For the HPLC measurement, much more enzyme was used compared to the samples that were prepared for the GC measurement. This shows that it is possible to influence the conversion rate with the proportion of enzyme to substrate.

### 7. Hydrogel

According to a preferred embodiment, the compositions according to the present inventions are provided as hydrogels. This involves the inclusion of another abuse deterrent approach, namely to create a system which changes the rheological properties of the reaction solution, i.e. the formation of a hydrogel. Hydrogels are very viscous and as a consequence, the drawing up with a needle is not possible. For this purpose different set ups were tried as shown in Table 7. The substances were mixed together until a gel was formed.

The first trial with chitosan formed a hydrogel after approx. 15 minutes. The idea is that catechol crosslinks the chitosan molecules and that the morphine is covalently imbedded via Michael's type reactions. This trial was a successful proof of concept while catechol needs to be replaced with other molecules already used as drug additives.

The second trial with carboxymethylchitosan formed a hydrogel just with morphine after approx. 24h. This system would be non-harmful and the reaction time can be optimized.

**Table 7: Conditions for enzymatic crosslinked hydrogels**

| Substances | Viscosity increased |
|---|---|
| Chitosan 2% (w/v) + 500 µM Catechol + morphine 10 mg/ml + 2U/ml MtL | + (after 15 min) |
| Carboxymethylchitosan 2% (w/v) + morphine 10mg/ml + 2U MtL | + (after 24 hours) |

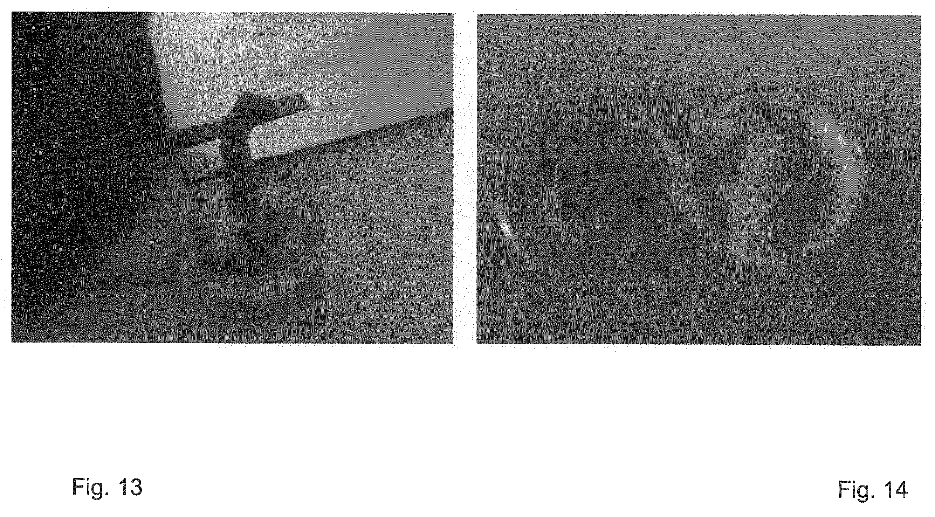

Fig. 13 shows the chitosan-catechol-morphine hydrogel Figure 14 shows the carboxymethylchitosan-morphine hydrogel. Both hydrogels are not injectable again and cannot be used anymore for administration.

### Discussion:

This study demonstrates an entirely new enzymatic approach for the development of abuse deterrent opioids. Tapentadol and morphine were successfully converted by the enzyme laccase which was confirmed by oxygen consumption measurements, TLC, HPLC-MS, FTIR and GC-MS analysis.

Since the enzymatic conversion of morphine was quite straight forward, this reaction was chosen as a model for further analysis. The reaction rate of the laccase from Myceliophthora thermophila on morphine was analysed via HPLC and GC measurement. After approx. 15 minutes 100% of morphine was converted to a product which precipitated. The precipitated reaction product was analysed via FTIR and also solubility tests were conducted. The precipitate was hardly soluble in any of the used solvents, which is desired for abuse prevention.

Overall the desired abuse prevention system based on enzyme polymerization was successfully developed. According to the presented results it is plausible that the present system is extendable in principle to all drugs, especially all opioids that have a laccase-reactive functional group.

### Preferred embodiments:

The present invention therefore relates to the following preferred embodiments:
1. Pharmaceutical composition comprising a drug with a laccase-reactive functional group and a laccase (EC 1.10.3.2).
2. Pharmaceutical composition according to embodiment 1, wherein the drug with a laccase-reactive functional group is selected from the group comprising substances with a phenolic hydroxyl-group, aminophenols, benzenethiols, di- or polyphenols, methoxy-substituted phenols, amino phenols, diamines, aromatic amines, polyamines, ascorbate, hydroxyindoles, aryl diamines, and anilins, preferably substances with a phenolic hydroxyl-group.
3. Pharmaceutical composition according to embodiment 1 or 2, wherein the laccase is selected from the group of laccase from Trametes villosa, laccase from Myceliophthora thermophila, and laccase from Pleurotus ostreatus.
4. Pharmaceutical composition according to any one of embodiments 1 to 3, wherein the drug is an opioid drug with a laccase-reactive functional group, preferably selected from the group morphine, tapentadol, hydromorphone, etorphine, desomorphine, oxymorphone, buprenorphine, opioid peptides comprising a phenylalanine residue, such as adrenorphin, amidorphin, casomorphin, DADLE ([D-Ala², D-Leu⁵]-Enkephalin), DAMGO ([D-Ala², N-MePhe⁴, Gly-ol]-enkephalin), dermorphin, endomorphin, morphiceptin, and TRIMU 5 (L-tyrosyl-N-{[(3-methylbutyl)amino]acetyl}-D-alaninamide); oripavine, 6-MDDM (6-methylenedihydrodesoxymorphine), chlornaltrexamine, dihydromorphine, hydromorphinol, methyldesorphine, N-phenethylnormorphine, RAM-378 (7,8-Dihydro-14-hydroxy-N-phenethylnormorphine), heterocodeine, 7-spiroindanyloxymorphone, morphinone, pentamorphone, semorphone, chloromorphide, nalbuphine, oxymorphazone, 1-iodomorphine, morphine-6-glucuronide, 6-monoacetylmorphine, normorphine, morphine-N-oxide, cyclorphan, dextrallorphan, levorphanol, levophenacylmorphan, norlevorphanol, oxilorphan, phenomorphan, furethylnorlevorphanol, xorphanol, butorphanol, 6,14-endoethenotetrahydrooripavine, BU-48 (N-Cyclopropylmethyl-[7α,8α,2',3']-cyclohexano-1'[S]-hydroxy-6,14-endo-ethenotetrahydronororipavine), cyprenorphine, dihydroetorphine, norbuprenorphine, 5'-guanidinonaltrindole, diprenorphine, levallorphan, meptazinol, methylnaltrexone, nalfurafine, nalmefene, naloxazone, naloxone, nalorphine, naltrexone, naltriben, naltrindole, 6β-naltrexol-d4, pseudomorphine, naloxonazine, norbinaltorphimine, alazocine, bremazocine, dezocine, ketazocine, metazocine, pentazocine, phenazocine, cyclazocine, hydroxypethidine (bemidone), ketobemidone, methylketobemidone, propylketobemidone, alvimopan, picenadol and pharmaceutically acceptable salts, hydrates, solvates, esters, prodrugs and mixtures thereof, more preferred morphine, tapentadol, buprenorphine, oxymorphone, pentazocine, levorphanol, hydromorphone, especially morphine.
5. Pharmaceutical composition according to any one of embodiments 1 to 4, wherein the pharmaceutical composition is selected from a tablet, a coat-core tablet, a bi-layer tablet, a multi-layer tablet, a capsule, a pellet, a MUPS (multiple unit pellet system), a granulate, a powder, especially coated, sugar-coated and/or enteric coated forms thereof.
6. Pharmaceutical composition according to any one of embodiments 1 to 5, wherein the composition comprises a coated laccase.
7. Pharmaceutical composition according to any one of embodiments 1 to 6, wherein the drug is contained in an amount of 0.1 to 5.000 mg, preferably 0.5 to 1.000 mg, especially 1 to 500 mg, per dosage unit.
8. Pharmaceutical composition according to any one of embodiments 1 to 7, wherein the laccase is contained in an amount of 1 to 1.000 units, preferably 10 to 100 units.
9. Pharmaceutical composition according to any one of embodiments 1 to 8, wherein the composition comprises a further abuse-deterrent feature, preferably selected from the group a physical or chemical barrier, especially increased tablet hardness, a drug antagonist, an aversion component, an abuse-deterrent delivery system and a prodrug, especially a physical barrier or an aversion component, especially a gelling agent and/or a non-gelling viscosity-increasing agent.
10. Pharmaceutical composition according to any one of embodiments 1 to 9, wherein the composition comprises a matrix containing 1 to 80 wt.% of one or more hydrophobic or hydrophilic polymers, preferably a matrix comprising agar, alamic acid, alginic acid, carmellose, carboxymethylcellulose sodium, carbomer, carrageenan, chitosan, especially carboxymethylchitosan, catechol , copovidone, dextrin, gelatin, guar gum, hydroxyethyl cellulose, hydroxypropyl cellulose, hydroxypropyl methylcellulose, methacrylic acid copolymers, methylcellulose derivatives, microcrystalline cellulose, polyacrylic acid, polyalkylene oxide, especially polyethylene glycol, polyvinyl alcohol, polyvinyl acetate, povidone, propylene glycol alginate, a polyvinylcaprolactam-polyvinyl acetate-polyethylene glycol graft co-polymer, pullulan, silicon dioxide, sodium alginate, starch, vinylpyrrolidone-vinyl acetate copolymers, xanthan gum; or of a non-polymer matrix former, preferably microcrystalline wax, fatty alcohols and fatty acids, especially stearyl alcohol, cetyl stearyl alcohol, stearic acid, palmitic acid or salts and mixtures thereof, mono-, di- and triglycerides of saturated fatty acids with a chain length between 16 and 22 carbon atoms and a mixture of such mono- di- and triglycerides.
11. Pharmaceutical composition according to any one of embodiments 1 to 10, wherein the composition is storage stable, preferably by comprising less than 5%, especially less than 1%, laccase-processed drug after 6 month storage at 25°C under dry conditions.
12. Pharmaceutical composition according to any one of embodiments 1 to 11, further comprising a hydrogel-forming component and/or a crosslinker, preferably chitosan and/or catechol or carboxymethylchitosan and/or vanillin.
13. Pharmaceutical composition according to any one of embodiments 1 to 12, wherein the composition is a modified release composition, especially a prolonged release composition.
14. Pharmaceutical composition according to any one of embodiments 1 to 13, wherein the composition renders immediate release, modified release, or a combination thereof.
15. Pharmaceutical composition according to any one of embodiments 1 to 14, wherein the composition comprises an opioid analgesic alone or in combination with a non-opioid analgesic, especially with ibuprofen, diclofenac, naproxen, paracetamol and acetyl-salicylic acid.
16. Pharmaceutical composition according to any one of embodiments 1 to 15 for use in the treatment of drug addiction.
17. Pharmaceutical composition according to any one of embodiments 1 to 15 for use in the treatment of pain.
18. Pharmaceutical composition according to any one of embodiments 1 to 15, wherein the drug-processing enzyme essentially does not act on the drug in vivo when the composition is swallowed intact.
19. Pharmaceutical composition according to any one of embodiments 1 to 15, wherein the drug-processing enzyme exists in the formulation in an essentially non-releasable form when the composition is swallowed intact.
20. Pharmaceutical composition according to any one of embodiments 1 to 15, wherein the drug-processing enzyme is deactivated in vivo.
21. Method for manufacturing a pharmaceutical composition according to any one of embodiments 1 to 20 comprising the steps of mixing the drug with the laccase and finishing the mixture to a pharmaceutical composition.
22. Method for manufacturing a pharmaceutical composition according to any one of embodiments 1 to 20 comprising the steps of providing the drug and the laccase in separated form and finishing the separated drug and laccase to a pharmaceutical composition.

## Claims

1. Pharmaceutical composition comprising a drug with a laccase-reactive functional group and a laccase (EC 1.10.3.2).

2. Pharmaceutical composition according to claim 1, wherein the drug with a laccase-reactive functional group is selected from the group comprising substances with a phenolic hydroxyl-group, aminophenols, benzenethiols, di- or polyphenols, methoxy-substituted phenols, amino phenols, diamines, aromatic amines, polyamines, ascorbate, hydroxyindoles, aryl diamines, and anilins" preferably substances with a phenolic hydroxyl-group.

3. Pharmaceutical composition according to claim 1 or 2, wherein the laccase is selected from the group of laccase from Trametes villosa, laccase from Myceliophthora thermophila, and laccase from Pleurotus ostreatus.

4. Pharmaceutical composition according to any one of claims 1 to 3, wherein the drug is an opioid drug with a laccase-reactive functional group, preferably selected from the group morphine, tapentadol, hydromorphone, etorphine, desomorphine, oxymorphone, buprenorphine, opioid peptides comprising a phenylalanine residue, such as adrenorphin, amidorphin, casomorphin, DADLE ([D-Ala², D-Leu⁵]-Enkephalin), DAMGO ([D-Ala², N-MePhe⁴, Gly-ol]-enkephalin), dermorphin, endomorphin, morphiceptin, and TRIMU 5 (L-tyrosyl-N-{[(3-methylbutyl)amino]acetyl}-D-alaninamide); oripavine, 6-MDDM (6-methylenedihydrodesoxymorphine), chlornaltrexamine, dihydromorphine, hydromorphinol, methyldesorphine, N-phenethylnormorphine, RAM-378 (7,8-Dihydro-14-hydroxy-N-phenethylnormorphine), heterocodeine, 7-spiroindanyloxymorphone, morphinone, pentamorphone, semorphone, chloromorphide, nalbuphine, oxymorphazone, 1-iodomorphine, morphine-6-glucuronide, 6-monoacetylmorphine, normorphine, morphine-N-oxide, cyclorphan, dextrallorphan, levorphanol, levophenacylmorphan, norlevorphanol, oxilorphan, phenomorphan, furethylnorlevorphanol, xorphanol, butorphanol, 6,14-endoethenotetrahydrooripavine, BU-48 (N-Cyclopropylmethyl-[7α,8α,2',3']-CyClohexano-1'[S]-hydroxy-6,14-endo-ethenotetrahydronororipavine), cyprenorphine, dihydroetorphine, norbuprenorphine, 5'-guanidinonaltrindole, diprenorphine, levallorphan, meptazinol, methylnaltrexone, nalfurafine, nalmefene, naloxazone, naloxone, nalorphine, naltrexone, naltriben, naltrindole, 6β-naltrexol-d4, pseudomorphine, naloxonazine, norbinaltorphimine, alazocine, bremazocine, dezocine, ketazocine, metazocine, pentazocine, phenazocine, cyclazocine, hydroxypethidine (bemidone), ketobemidone, methylketobemidone, propylketobemidone, alvimopan, picenadol and pharmaceutically acceptable salts, hydrates, solvates, esters, prodrugs and mixtures thereof, more preferred morphine, tapentadol, buprenorphine, oxymorphone, pentazocine, levorphanol, hydromorphone, especially morphine.

5. Pharmaceutical composition according to any one of claims 1 to 4, wherein the pharmaceutical composition is selected from a tablet, a coat-core tablet, a bi-layer tablet, a multi-layer tablet, a capsule, a pellet, a MUPS (multiple unit pellet system), a granulate, a powder, especially coated, sugar-coated and/or enteric coated forms thereof.

6. Pharmaceutical composition according to any one of claims 1 to 5, wherein the composition comprises a coated laccase.

7. Pharmaceutical composition according to any one of claims 1 to 6, wherein the drug is contained in an amount of 0.1 to 5.000 mg, preferably 0.5 to 1.000 mg, especially 1 to 500 mg, per dosage unit.

8. Pharmaceutical composition according to any one of claims 1 to 7, wherein the laccase is contained in an amount of 1 to 1.000 units, preferably 10 to 100 units.

9. Pharmaceutical composition according to any one of claims 1 to 8, wherein the composition comprises a further abuse-deterrent feature, preferably selected from the group a physical or chemical barrier, especially increased tablet hardness, a drug antagonist, an aversion component, an abuse-deterrent delivery system and a prodrug, especially a physical barrier or an aversion component, especially a gelling agent and/or a non-gelling viscosity-increasing agent.

10. Pharmaceutical composition according to any one of claims 1 to 9, wherein the composition comprises a matrix containing 1 to 80 wt.% of one or more hydrophobic or hydrophilic polymers, preferably a matrix comprising agar, alamic acid, alginic acid, carmellose, carboxymethylcellulose sodium, carbomer, carrageenan, chitosan, especially carboxymethylchitosan, catechol , copovidone, dextrin, gelatin, guar gum, hydroxyethyl cellulose, hydroxypropyl cellulose, hydroxypropyl methylcellulose, methacrylic acid copolymers, methylcellulose derivatives, microcrystalline cellulose, polyacrylic acid, polyalkylene oxide, especially polyethylene glycol, polyvinyl alcohol, polyvinyl acetate, povidone, propylene glycol alginate, a polyvinylcaprolactam-polyvinyl acetate-polyethylene glycol graft co-polymer, pullulan, silicon dioxide, sodium alginate, starch, vinylpyrrolidone-vinyl acetate copolymers, xanthan gum; or of a non-polymer matrix former, preferably microcrystalline wax, fatty alcohols and fatty acids, especially stearyl alcohol, cetyl stearyl alcohol, stearic acid, palmitic acid or salts and mixtures thereof, mono-, di- and triglycerides of saturated fatty acids with a chain length between 16 and 22 carbon atoms and a mixture of such mono- di- and triglycerides.

11. Pharmaceutical composition according to any one of claims 1 to 10, wherein the composition is storage stable, preferably by comprising less than 5%, especially less than 1%, laccase-processed drug after 6 month storage at 25°C under dry conditions.

12. Pharmaceutical composition according to any one of claims 1 to 11, further comprising a hydrogel-forming component and/or a crosslinker, preferably chitosan and/or catechol or carboxymethylchitosan and/or vanillin.

13. Method for manufacturing a pharmaceutical composition according to any one of claims 1 to 12 comprising the steps of mixing the drug with the laccase and finishing the mixture to a pharmaceutical composition.

14. Method for manufacturing a pharmaceutical composition according to any one of claims 1 to 12 comprising the steps of providing the drug and the laccase in separated form and finishing the separated drug and laccase to a pharmaceutical composition.

15. Pharmaceutical composition according to any one of claims 1 to 12 for use in the treatment of pain and/or drug addiction.
